# EUROPEAN PATENT SPECIFICATION

(11) **EP 0 614 870 B1**
(45) Date of publication and mention of the grant of the patent: **14.01.1998**
(21) Application number: 94103675.8
(22) Date of filing: 10.03.1994
(51) Int. Cl.: C07C 45/50, C07C 45/49, C07C 47/14, C07C 47/228, C07C 47/277, C07C 67/293, C07C 69/14, C07D 209/48, C07C 253/30, C07C 255/17

(54) **Process for producing optically active aldehydes**
Verfahren zur Herstellung von optisch aktiven Aldehyden
Procédé pour la préparation d'aldéhydes optiquement actifs

(30) Priority: 12.03.1993 JP 52539/93
(43) Date of publication of application: 14.09.1994
(73) Proprietor: MITSUBISHI GAS CHEMICAL COMPANY, INC., Minato-ku, Tokyo (JP); Takasago International Corporation, Tokyo 108 (JP)
(72) Inventor: Takaya, Hidemasa, Kusatsu-shi, Shiga (JP); Sakai, Nozomu, Kobe-shi, Hyogo (JP); Tamao, Kyoko, Beru Mezon Kamogawa 303, Kamikyo-ku, Kyoto-shi, Kyoto (JP); Mano, Satoshi, Ichinomiya-shi, Aichi (JP); Kumobayashi, Hidenori, c/o Takasago Int. Corp., Hiratsuka-shi, Kanagawa (JP); Tomita, Tetsuo, Katsushika-ku, Tokyo (JP)
(74) Representative: Kraus, Walter, Dr.

(56) References cited:
- WO-A-93/03839
- TETRAHEDRON: ASYMMETRY, vol.3, no.5, 1992, OXFORD GB pages 583 - 586 N SAKAI ET AL
- JOURNAL OF THE CHEMICAL SOCIETY, CHEMICAL COMMUNICATIONS, no.4, 21 February 1994, LETCHWORTH GB pages 395 - 396 N SAKAI ET AL
- JOURNAL OF THE AMERICAN CHEMICAL SOCIETY, vol.115, no.15, 28 July 1993, WASHINGTON, DC US pages 7033 - 7034 N SAKAI ET AL

## Description

The present invention relates to a process for producing optically active aldehydes, and more particularly to a process for producing an optically active aldehyde represented by the following formula (2) using a vinyl compound represented by the following formula (1), carbon monoxide and hydrogen as starting materials.

CH₂=CH-Q (1)

OHC-CH(CH₃)-Q (2)

wherein Q means a halogen atom, lower alkyl group, phthalimide group, lower alkylcarbonyloxy group, cyano group, carboxyl group, lower alkyloxycarbonyl group, lower alkoxy group, lower alkylcarbonyl group, acetylamino group, benzoylamino group, monoalkylamino group, dialkylamino group, benzoyl group, phenyl group which may be substituted by a halogen atom, lower alkyl group or lower alkoxy group, or naphthyl group which may be substituted by a halogen atom, lower alkyl group or lower alkoxy group.

Asymmetric hydroformylation reactions have heretofore attracted much attention in the field of synthetic organic chemistry because of their good practical utility upon synthesis of optically active aldehydes. In particular, in order to achieve both high regioselectivity (ratio of branched chain to linear chain) and high stereoselectivity in a step wherein a carbon-carbon bond is formed, a great deal of effort has been given to the development of a effective catalyst for asymmetric synthesis. For example, rhodium or platinum complexes modified by an optically active diphosphine have been used as catalysts.

However, results obtained to date have been not satisfactory in many cases from the viewpoint of synthetic chemistry. When the platinum catalyst is used in an asymmetric hydroformylation reaction, its reaction rate is generally slow, and besides there is a tendency for the hydrogenation reaction of a starting material to competitively progress. The use of the rhodium catalyst solves such drawbacks. However, such a catalyst does not entirely have no problems.

There have been several attempts to asymmetrically hydroformylate vinyl acetate to obtain optically active 2-acetoxypropanal. For example, according to C. F. Hobbs and W. S. Knowles, J. Org. Chem., 46, 4422 (1981), vinyl acetate is asymmetrically hydroformylated using a mixture of DIPHOL and Rh(cod)(acac) as a catalyst to achieve an optical purity of 51% ee at the highest.

The present inventors effected an asymmetric hydroformylation reaction of vinyl acetate using a rhodium catalyst containing, as a ligand, bis(triarylphosphite) which is easier to synthesize than DIPHOL and reported it in Tetrahedron Asymmetry, 3(5), 583 (1992). According to this process, practical utility in industry was greatly improved, but its optical purity was 49% ee and hence differed little from that of the conventional process.

Besides, an example of the asymmetric hydroformylation reaction of olefins making use of a rhodium complex containing an optically active bidentate phosphine (DIOP or the like) as a ligand is described in Bull. Chem. Soc. Japan 52, 2605 (1979), and an example of the asymmetric hydroformylation reaction of methyl α-acetamidoacrylate making use of a rhodium complex containing an optically active bidentate phosphine as a ligand is described in Tetrahedron Asymmetry, 10, 693 (1990). In each case, the optical purity is not high.

In the hydroformylation reaction, good yield can be generally achieved using a cobalt or rhodium catalyst. However, various means have been made for enhancing the selectivity of a position to which a formyl group adds. The positional selectivity has become very high by methods such as the addition of a phosphine ligand. However, it has been difficult to furthermore stereospecifically control the hydroformylation, and the optical purity of an aldehyde produced has been up to 50% ee at most.

In order to enhance steric selectivity, it is necessary to strictly control a configuration space about a center metal of a catalyst, thereby limiting the route of the addition of the formyl group in one direction on the configuration space. Such control of the configuration space can be attained only by satisfactorily designing the steric configuration of the ligand. It is apparent that the catalysts conventionally used are insufficient in controlling the configuration space. Therefore, the present inventors intended to design a ligand using an optically active binaphthol which is a far excellent asymmetry source and recently came to be available with ease. For such an end, bis(triarylphosphite) ligands were already synthesized and tested. However, unsatisfactory results were only obtained.

In view of the foregoing circumstances, the present inventors have carried out an extensive investigation with a view toward solving the above-described drawbacks. As a result, it has been surprisingly found that when a new phosphine compound having a phosphine structure and a phosphite structure at the same time in a binaphthyl skeleton is synthesized, and a catalyst obtained by using this phosphine compound and rhodium as a ligand and a center metal, respectively, is used to conduct the hydroformylation reactions of various vinyl compounds, stereoregularity is exhibited to a very high extent unexpectable from the conventionally used catalysts, thus leading to completion of the present invention.

In an aspect of the present invention, there is thus provided a process for producing an optically active aldehyde represented by the following formula (2):

OHC-CH(CH₃)-Q (2)

wherein Q means a halogen atom, C₁-C₆ alkyl group, phthalimide group, C₁-C₆ alkylcarbonyloxy group, cyano group, carboxyl group, C₁-C₆ alkyloxycarbonyl group, C₁-C₆ alkoxy group, C₁-C₆ alkylcarbonyl group, acetylamino group, benzoylamino group, monoalkylamino group, dialkylamino group, benzoyl group, phenyl group which may be substituted by a halogen atom, C₁-C₆ alkyl group or C₁-C₆ alkoxy group, or naphthyl group which may be substituted by a halogen atom, C₁-C₆ alkyl group or C₁-C₆ alkoxy group, which comprises hydroformylating a vinyl compound represented by the following formula (1):

CH₂=CH-Q (1)

wherein Q has the same meaning as defined above, using, as a catalyst, a rhodium-phosphine complex represented by the following formula (3-a) or (3-b):

[Rh(L)(Y)(X)] (3-a)

[Rh(L)(Y)](X) (3-b)

wherein L means a phosphine compound represented by the following formula (4): 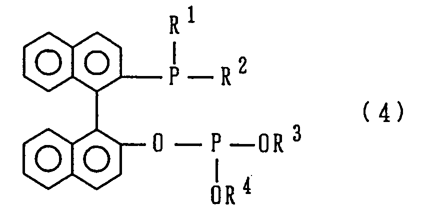 wherein R¹ and R² are identical with or different from each other and mean individually a phenyl group which may be substituted by a halogen atom or C₁-C₆ alkyl group, or denote together a divalent hydrocarbon group, R³ and R⁴ are identical with or different from each other and mean individually a C₁-C₆ alkyl group or a phenyl group which may be substituted by a halogen atom, C₁-C₆ alkyl group or C₁-C₆ alkoxy group, or denote together a divalent hydrocarbon group, Y denotes carbon monoxide, a monodentate or bidentate neutral ligand having lower coordinating strength than that of carbon monoxide, olefin or diene, and X stands for a hydrogen atom, monovalent anion or triphenylphosphine, or X and Y mean together a β-diketonate.

According to the present invention, optically active aldehydes which require much labor and time to synthesize, and are expensive can be produced at a high yield and a low cost by a simple process under mild conditions.

In particular, the process of this invention allows the reaction to progress in such a high optical purity as has been unable to be reached by any conventional asymmetric hydroformylation.

The above and other objects, features and advantages of the present invention will become apparent from the following description and the appended claims.

In the present invention, the term "C₁-C₆ alkyl group" means a linear or branched alkyl group having 1-6 carbon atoms, such as a methyl, ethyl, propyl, isopropyl, butyl, isobutyl, sec-butyl, tert-butyl, pentyl or hexyl group. The term "halogen atom" means a chlorine, bromine, iodine or fluorine atom, while the term "C₁-C₆ alkoxy group" means a linear or branched alkoxy group having 1-6 carbon atoms, such as a methoxy, ethoxy, propoxy, isopropoxy, butoxy, isobutoxy, sec-butoxy or tert-butoxy group. The C₁-C₆ alkyl groups in the C₁-C₆ alkylcarbonyloxy group, C₁-C₆ alkyloxycarbonyl group and C₁-C₆ alkylcarbonyl group also have the same meaning as described above. Further, the alkyl groups in the monoalkylamino group and dialkylamino group also have the same meaning as described above.

Besides, the term "divalent hydrocarbon group" as used herein means a biarylene group such as a biphenyl group which may have a substituent group, a binaphthyl group which may have a substituent group, a biphenanthryl group which may have a substituent group or a bianthryl group which may have a substituent group, or a linear or branched, saturated or unsaturated alkylene group having 2-7 carbon atoms, such as a tetramethylene, pentamethylene, 1,4-dimethyltetramethylene, 1,3-butadienylene or 1,4-dimethyl-1,3-butadienylene group. Examples of the substituent group substitutable on the bisarylene group include lower alkyl groups, halogen atoms and lower alkoxy groups.

The present invention is characterized by the following reaction formula: 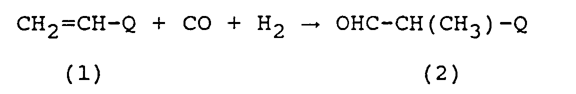 wherein Q has the same meaning as defined above.

The catalyst useful in the practice of this invention is represented by the following formula (3-a) or (3-b):

[Rh(L)(Y)(X)] (3-a)

[Rh(L)(Y)](X) (3-b)

wherein L, X and Y have the same meaning as defined above.

The phosphine compound can be synthesized at a high yield in accordance with the following reaction scheme: 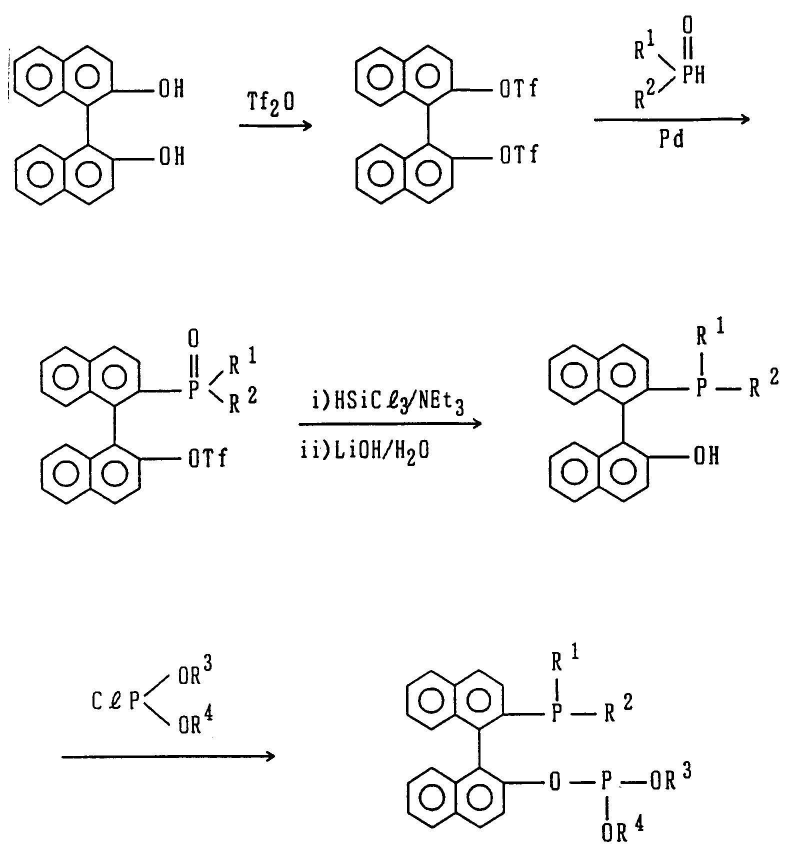 wherein Tf means a trifluoromethanesulfonyl group, and R¹, R², R³ and R⁴ have the same meaning as defined above.

The phosphine compound thus synthesized was stable in both air and moist air and could be simply purified by column chromatography on silica gel. The values of elemental analysis and spectral data of this compound showed that it surely has a structure of phosphine compound. Its HPLC analysis proved that the compound is optically pure.

The rhodium-phosphine complex useful in the practice of this invention can be synthesized at a good yield from a rhodium complex such as [Rh(CO)₂Cl]₂ and the phosphine compound according to the present invention.

As the rhodium compound, may be used rhodium metal complexes, mineral acid rhodium salts, organic acid rhodium salts, rhodium oxides and the like. Among a variety of these rhodium compounds, rhodium complexes such as rhodium triacetylacetonate, rhodium dicarbonylacetylacetonate, tetrarhodium dodecacarbonyl, hexarhodium hexadecacarbonyl and rhodium cyclooctadieneacetylacetonate, rhodium nitrate, rhodium sulfate, rhodium acetate and rhodium oxides are preferred. Incidentally, the rhodium-phosphine complex is generally a mononuclear complex in which one rhodium atom is contained in the complex. However, there may be polynuclear complexes containing 2 or more rhodium atoms therein in some cases.

Examples of the vinyl compound which is a substrate in the present invention include vinyl chloride, vinyl bromide, vinyl iodide, 1-propene, 1-butene, 1-pentene, 1-hexene, 3,3-dimethyl-1-butene, N-vinylphthalimide, vinyl acetate, vinyl propionate, vinyl valerate, acrylonitrile, acrylic acid, methyl acrylate, ethyl acrylate, butyl acrylate, tert-butyl acrylate, methyl vinyl ether, ethyl vinyl ether, butyl vinyl ether, tert-butyl vinyl ether, 3-butene-2-on, 1-heptene-3-on, 4,4-dimethyl-1-pentene-3-on, N-vinylacetamide, N-vinylbenzamide, methylvinylamine, ethylvinylamine, butylvinylamine, dimethylvinylamine, diethylvinylamine, dibutylvinylamine, styrene, chlorostyrene, bromostyrene, methylstyrene, 4-tert-butylstyrene, 4-isobutylstyrene, methoxystyrene, ethoxystyrene, vinylnaphthalene, 2-methoxy-6-vinylnaphthalene, 1-chloro-1-propene and 1-bromo-1-propene.

In the process of this invention, it may be permissible to either add the rhodium-phosphine complex, or separately add the rhodium compound and the phosphine compound to the reaction system to form the rhodium-phosphine complex in the system. With respect to the concentration of the catalyst in the reaction system, it is used in an amount corresponding to a range of 0.0001-1,000 milligram atom, preferably 0.001-100 milligram atom in terms of rhodium atom, per liter of a liquid phase.

Although the process of the present invention may be effected without using any reaction solvent, it is generally preferable to conduct a reaction in the presence of a reaction solvent. Any reaction solvents may be used so far as they do not adversely affect the reaction. However, hydrocarbons are particularly preferred. As specific examples thereof, may be mentioned hexane, heptane, octane, nonane, decane, benzene, toluene, xylene and the like. Besides, preferable examples thereof include ethers such as dipropyl ether, dibutyl ether, tetrahydrofuran and dimethoxyethane, ketones such as diisobutyl ketone and methyl isobutyl ketone, and esters such as butyl butyrate and butyl benzoate.

In the hydroformylation reaction, a process in which water is caused to coexist in the reaction system is generally preferred for enhancing catalytic activity. In the process of the present invention, water may also be caused to coexist upon the reaction. No particular limitation is imposed on the amount of water to be added. However, the use of a too small amount of water has little effect. If a too large amount of water is used on the other hand, the effect is not heightened correspondingly. In general, when water is added in an amount 0.001 to 1,000 times by weight of the substrate vinyl compound, the reaction rate may be increased in some cases.

In the process of the present invention, various additives other than water may be added with a view toward improving the activity, positional selectivity and steric selectivity of the catalyst and more improving the stability of the catalyst. When one or more of phosphorus compounds such as phosphine compounds, and besides, triethylphosphine oxide, tributylphosphine oxide, triphenylphosphine oxide, triethylphosphite, tributylphosphite and triphenylphosphite, nitrogen compounds such as fatty acid amines, aromatic amines, pyridine compounds, quinoline compounds, imidazole compounds, diamines, triamines, aminoalcohols and amides, and carboxylic acids such as acetic acid, propionic acid and pivalic acid are caused to coexist, the activity and the like of the catalyst may be improved in some cases.

With respect to reaction conditions upon effecting the process of this invention, the reaction temperature may preferably be within a range of from -20 to 250°C, more preferably from 10 to 150°C. A low reaction temperature is preferred from the viewpoint of the heat stability of aldehydes formed, while a high reaction temperature is desired from the viewpoint of the reaction rate. The reaction may preferably be conducted under a pressure ranging from 4.9 to 196.2 bar (5 to 200 kp/cm²), more preferably from 19.6 to 147 bar (20 to 150 kp/cm²). In general, the mixing molar ratio of carbon monoxide to hydrogen as raw materials may preferably be within a range of from 10 to 0.1, more preferably from 4 to 0.2. These gases may be diluted with other gases inert to the reaction so far as the mixing ratio of carbon monoxide to hydrogen is maintained to such a range. As the dilute gases, methane, nitrogen, argon, helium, carbon dioxide and the like may be used either singly or in any combination thereof.

The process of the present invention can be conducted by any one of batch, semi-batch and continuous processes. If the reaction is conducted by, for example, the batch process, predetermined amounts of the rhodium-phosphine complex and vinyl compound, and optionally the reaction solvent and additives are charged in an autoclave, in which a mixed gas containing carbon monoxide and hydrogen is introduced to a predetermined pressure. While stirring the contents, they are then heated to a predetermined temperature, whereby the reaction is allowed to progress. In the case of the continuous process, the rhodium-phosphine complex, vinyl compound and additives are dissolved in the solvent. The resulting solution is continuously fed from one side of a pressure reactor by means of a stroke pump, and at the same time, a mixed gas of carbon monoxide and hydrogen is supplied to maintain the reaction pressure constant. The reaction is conducted while stirring the contents under heat, and at the same time, parts of the liquid reaction mixture and mixed gas are continuously drawn out from the other side of the reactor.

The process of the present invention will hereinafter be described more specifically by the following examples.

### Example 1:

### Synthesis of (R)-2,2'-bis(trifluoromethanesulfonyloxy)-1,1'-binaphthyl (hereinafter called "Compound I"):

In a 100-ml flask, 5 g (17.4 mmol) of (R) -1,1'-bi-2-naphthol, 5.58 g (52.2 mmol) of 2,6-lutidine and 0.955 g (7.83 mmol) of 4-dimethylaminopyridine were dissolved in 26 ml of methylene chloride, to which 14.7 g (52.2 mmol) of trifluoromethanesulfonic anhydride was added at 0°C. The contents were stirred for 23 hours at room temperature to complete a reaction. After completion of the reaction, the solvent was distilled off, and the residue was then purified by column chromatography on silica gel making use of methylene chloride as a developing solvent to obtain 9.56 g (yield: 100%) of (R)-2,2'-bis(trifluoromethanesulfonyloxy)-1,1'-binaphthyl.

### Synthesis of (R)-2-diphenylphosphinyl-2'-trifluoromethanesulfonyloxy-1,1'-binaphthyl (hereinafter called "Compound II"):

In a 100-ml 4-necked flask, 2.74 g (4.98 mmol) of Compound I and 1.99 g (9.82 mmol) of diphenylphosphine oxide were dissolved in 20 ml of dimethyl sulfoxide in an argon stream, to which 110 mg (0.491 mmol) of palladium acetate, 203 mg (0.491 mmol) of 1,3-bis(diphenylphosphino)propane, 5.1 ml of ethyldiisopropylamine and 33 mg (0.491 mmol) of sodium formate were added to stir them for 20 minutes at room temperature. Thereafter, the resultant solution was stirred for 19 hours at 90°C and then cooled back to room temperature. The solution was added with 250 ml of ether and 150 ml of water, and the mixture was stirred to separate an organic layer and an aqueous layer. After the separation, the organic layer was washed four times with 125 ml of water, twice with 125 ml of 5% diluted hydrochloric acid, twice with 50 ml of water, once with 125 ml of a saturated aqueous solution of sodium hydrogencarbonate and lastly with 125 ml of a brine. The thus-washed organic layer was dried on anhydrous magnesium sulfate, and the solvent was distilled off. The residue was then purified by column chromatography on silica gel making use of a 3:1 (solvent ratio, the same shall apply hereinafter) mixture of toluene and acetonitrile as a developing solvent, thereby obtaining 2.51 g (yield: 83%) of the title compound.

### Synthesis of (R)-2-diphenylphosphino-2'-hydroxy-1,1'-binaphthyl (hereinafter called "Compound III"):

In a 50-ml 3-necked flask, 400 mg (0.664 mmol) of Compound II was dissolved in 22 ml of xylene, to which 1.21 g (12 mmol) of triethylamine and 1.62 g (12 mmol) of trichlorosilane were added. The resulting mixture was stirred for 17 hours at 120°C. After the reaction mixture was cooled back to room temperature, 4.4 ml of 35% aqueous sodium hydroxide was carefully added thereto, and the mixture was stirred further for 2 hours, followed by separation of an organic layer and an aqueous layer. After the separation, the organic layer was washed twice with 30 ml of a brine and then dried over anhydrous magnesium sulfate, and the solvent was distilled off under reduced pressure, thereby obtaining a crude product. The crude product was dissolved in 7 ml of tetrahydrofuran, to which a solution of 335 mg (7.98 mmol) of lithium hydroxide in 2.4 ml of water was added. The resulting mixture was stirred for 15 hours at room temperature, to which 50 ml of ether and 15 ml of 5% diluted hydrochloric acid were added to separate an organic layer and an aqueous layer. After the organic layer was washed twice with water, it was dried over anhydrous magnesium sulfate, and the solvent was distilled off. The residue was then purified by column chromatography on silica gel making use of a 5:1 mixture of hexane and ethyl acetate as a developing solvent, thereby obtaining 153 mg (yield: 51%) of the title compound.

### Synthesis of (S)-1,1'-binaphthalene-2,2'-diyldioxychlorophosphine (hereinafter called "Compound IV"):

A 50-ml flask was charged with 4.94 g (17.3 mmol) of (S)-1,1'-bi-2-naphthol and 113 g (0.83 mol) of phosphorus trichloride in an argon stream to reflux the mixture for 4 hours. Thereafter, the reaction mixture was cooled back to room temperature, and unreacted phosphorus trichloride was distilled off under reduced pressure, thereby obtaining 5.56 g (yield: 92%) of the title compound as colorless crystals.

### Synthesis of (R)-2-diphenylphosphino-1,1'-binaphthalene-2'-yloxy((S)-1,1'-binaphthalene-2,2'-diyldioxy)-phosphine [hereinafter abbreviated as "(R,S)-BINAPHOS"]:

In a 100-ml flask, 430 mg (0.946 mmol) of Compound III and 662 mg (1.89 mmol) of Compound IV were dissolved in 30 ml of ether, to which 191 mg (1.89 mmol) of triethylamine was added at 0°C. After the mixture was stirred for 15 hours at room temperature, 20 ml of water was added to stop the reaction. After separating an organic layer and an aqueous layer, the organic layer was dried on anhydrous magnesium sulfate, and the solvent was distilled off, thereby obtaining a crude product. The crude product was purified by column chromatography on silica gel making use of a 1:1 mixture of hexane and dichloromethane as a developing solvent, thereby obtaining 712 mg (yield: 98%) of (R,S)-BINAPHOS as white crystals. ³¹P-NMR (CDCl₃) δ: -14.6(d,J=29.0Hz), 144.7(d,J=29.0Hz).

### Asymmetric hydroformylation reaction of vinyl acetate:

A 50-ml autoclave was charged with 1.301 g (15.13 mmol) of vinyl acetate, 9.8 mg (0.0378 mmol) of Rh(CO)₂(acac) as a catalyst precursor, 58 mg (0.0755 mmol) of (R,S)-BINAPHOS as an optically active phosphine and 14 ml of benzene. The contents were stirred at 60°C for 112 hours under a hydrogen pressure of 50 atm and a carbon monoxide pressure of 50 atm. The determination of conversion of vinyl acetate and the analysis of the reaction products were conducted by gas chromatography (10% SE-30 on Chromosorb W 80-100 mesh packed in a 5 mm x 2 m glass column), and the optical purities of the products were determined by gas chromatography (Astec Chiraldex B-PH). As a result, it was found that compounds produced by this reaction were a mixture of 90% of 2-acetoxypropanal and 10% of 3-acetoxypropanal, and the conversion was 98%. 2-Acetoxypropanal was then distilled to determine its optical purity. As a result, it was found to be 90% ee. This compound was then caused to undergo Jones oxidation to derive 2-acetoxypropionic acid. Its optical rotation was measured. As a result, it was confirmed that the compound is (S)-(-)-2-acetoxypropionic acid.

### Example 2:

### Synthesis of (R)-2-(diphenylphosphino)-1,1'-binaphthalene-2'-yloxy((R)-1,1'-binaphthalene-2,2'-diyldioxy)phosphine [hereinafter abbreviated as "(R,R)-BINAPHOS"]:

A 100-ml flask was charged with 30 ml of ether, to which 209 mg (0.46 mmol) of Compound III and 322 mg (0.92 mmol) of (R)-1,1'-binaphthalene-2,2'-diyldioxychlorophosphine were added to dissolve them in ether. To the solution, 191 mg (1.89 mmol) of triethylamine was added at 0°C. Thereafter, the same procedure as in (R,S)-BINAPHOS was followed to obtain 353 mg (yield: 96.3%) of the title compound.
- ³¹P-NMR (CDCl₃) δ:: -18.0(d,J=9.2Hz), 140.5(d,J=9.2Hz).

### Synthesis of Rh((R,R)-BINAPHOS)(acac):

A 20-ml Schlenk's tube was charged 0.025 mmol of (R,R)-BINAPHOS and 6.5 mg (0.025 ml) of Rh(CO)₂(acac) (acac means acetylacetonate, the same shall apply hereinafter) to dissolve them in 5 ml of methylene chloride. After the reaction mixture was stirred for 5 minutes at room temperature, the solvent was distilled off under reduced pressure to obtain 63 mg (yield: 99%) of Rh((R,R)-BINAPHOS)(acac).
- ³¹P-NMR (CDCl₃) δ:: 51.9(dd,Jₚ₋ₚ=80.8Hz,J_{Rh-p}=178.4Hz), 152.5(dd,J_{Rh-p}=335.1Hz).

### Asymmetric hydroformylation reaction of vinyl acetate:

The same procedure as in Example 1 was followed except that Rh((R,R)-BINAPHOS)(acac) was used as a catalyst, and the reaction was conducted for 37 hours at 50°C, thereby obtaining a mixture of 92% of 2-acetoxypropanal and 8% of 3-acetoxypropanal. The conversion was 46%. The optical purity of 2-acetoxypropanal after distilled was determined in the same manner as in Example 1, and was found to be 73% ee. This compound was then caused to undergo Jones oxidation to derive 2-acetoxypropionic acid. Its optical rotation was measured. As a result, it was confirmed that the compound is (S)-(-)-2-acetoxypropionic acid.

### Example 3:

A 50-ml autoclave was charged with 3.06 g (29.4 mmol) of styrene, 3.7 mg (0.0143 mmol) of Rh(CO)₂(acac), 44 mg (0.0573 mmol) of (S,R)-BINAPHOS and 1 ml of benzene. The contents were stirred at 60°C for 43 hours under a hydrogen pressure of 50 atm and a carbon monoxide pressure of 50 atm. After completion of the reaction, the reaction mixture was cooled back to room temperature, and the gases under pressure were removed. Thereafter, the reaction mixture was directly analyzed by ¹H-NMR. As a result, it was found that the conversion of styrene was at least 99%, and a mixture of 88% of 2-phenylpropanal and 12 % of 3-phenylpropanal as reaction products was obtained in an amount of 1.273 g. No other products were observed. After distilling, the thus-obtained 2-phenylpropanal was caused to undergo Jones oxidation to derive 2-phenyl-propionic acid. Its optical purity was determined. As a result, it was confirmed that the compound is (S)-(+)-2-phenylpropionic acid having an optical purity of 94% ee.

### Examples 4-9:

The same procedure as in Example 1 was followed except that the substrate, the phosphine as a ligand, the amount of the rhodium compound, and the reaction temperature and time were changed as shown in Table 1, thereby obtaining results corresponding to the following reaction formula: 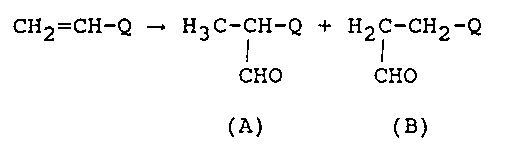 The results are shown in Table 1.

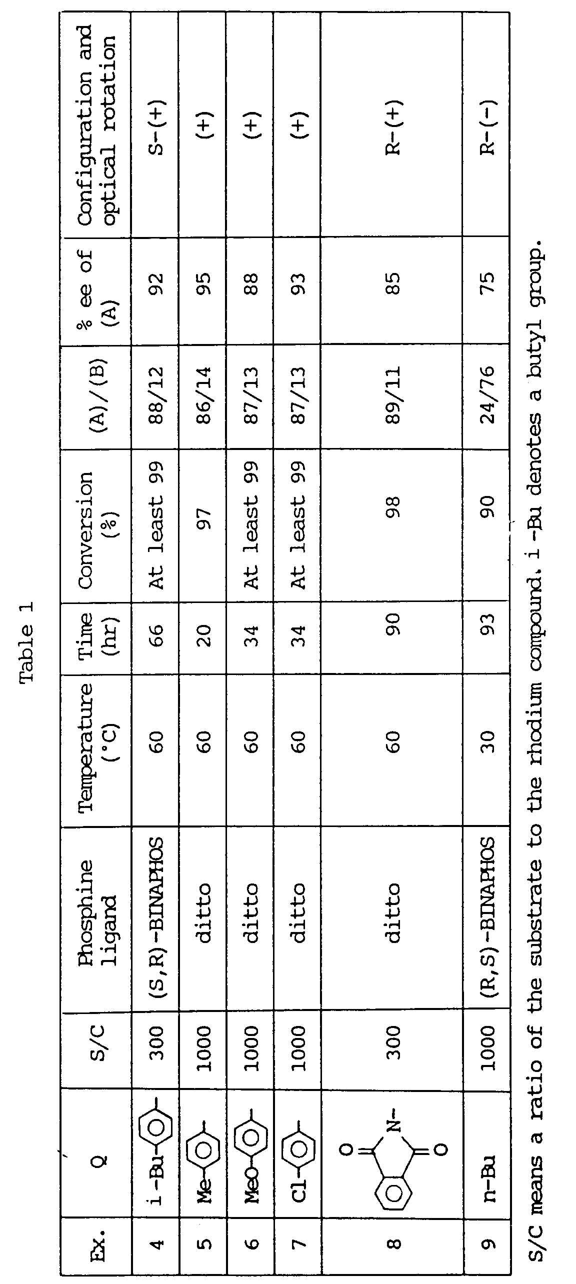

## Claims

1. A process for producing an optically active aldehyde represented by the following formula (2):
OHC-CH(CH₃)-Q (2)
wherein Q means a halogen atom, C₁-C₆ alkyl group, phthalimide group, C₁-C₆ alkylcarbonyloxy group, cyano group, carboxyl group, C₁-C₆ alkyloxycarbonyl group, C₁-C₆ alkoxy group, C₁-C₆ alkylcarbonyl group, acetylamino group, benzoylamino group, monoalkylamino group, dialkylamino group, benzoyl group, phenyl group which may be substituted by a halogen atom, C₁-C₆ alkyl group or C₁-C₆ alkoxy group, or naphthyl group which may be substituted by a halogen atom, C₁-C₆ alkyl group or C₁-C₆ alkoxy group, which comprises hydroformylating a vinyl compound represented by the following formula (1):
CH₂=CH-Q (1)
wherein Q has the same meaning as defined above, using, as a catalyst, a rhodium-phosphine complex represented by the following formula (3-a) or (3-b):
[Rh(L)(Y)(X)] (3-a)
[Rh(L)(Y)](X) (3-b)
wherein L means a phosphine compound represented by the following formula (4): 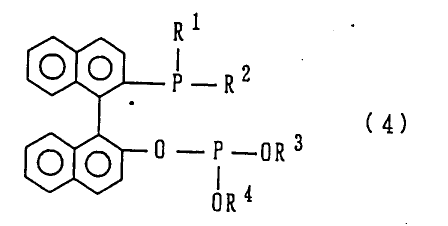 wherein R¹ and R² are identical with or different from each other and mean individually a phenyl group which may be substituted by a halogen atom or C₁-C₆ alkyl group, or denote together a divalent hydrocarbon group, R³ and R⁴ are identical with or different from each other and mean individually a C₁-C₆ alkyl group or a phenyl group which may be substituted by a halogen atom, C₁-C₆ alkyl group or C₁-C₆ alkoxy group, or denote together a divalent hydrocarbon group, Y denotes carbon monoxide, a monodentate or bidentate neutral ligand having lower coordinating strength than that of carbon monoxide, olefin or diene, and X stands for a hydrogen atom, monovalent anion or triphenylphosphine, or X and Y mean together a β-diketonate.

2. The process according to Claim 1, wherein the vinyl compound is selected from vinyl acetate, N-vinylphthalimide, vinyl chloride, styrene, acrylonitrile, 4-isobutylstyrene and 2-methoxy-6-vinylnaphthalene.

3. The process according to Claim 1, wherein the binaphthyl skeleton of the phosphine compound in the formula (3-a) or (3-b) has an optically active R or S structure.

## Patentansprüche

1. Verfahren zur Herstellung eines optisch aktiven Aldehyds, dargestellt durch die folgende Formel (2):
OHC-CH(CH₃)-Q (2)
worin Q ein Halogenatom, eine C₁-C₆-Alkylgruppe, Phthalimidgruppe, C₁-C₆-Alkylcarbonyloxygruppe, Cyanogruppe, Carboxylgruppe, C₁-C₆-Alkyloxycarbonylgruppe, C₁-C₆-Alkoxygruppe, C₁-C₆-Alkylcarbonylgruppe, Acetylaminogruppe, Benzoylaminogruppe, Monoalkylaminogruppe, Dialkylaminogruppe, Benzoylgruppe, Phenylgruppe, die durch ein Halogenatom, eine C₁-C₆-Alkylgruppe oder C₁-C₆-Alkoxygruppe substituiert sein kann, oder eine Naphthylgruppe, die durch ein Halogenatom, eine C₁-C₆-Alkylgruppe oder eine C₁-C₆-Alkoxygruppe substituiert sein kann, bedeutet, umfassend die Hydroformylierung einer Vinylverbindung, dargestellt durch die folgende Formel (1):
CH₂=CH-Q (1)
worin Q die gleiche Bedeutung, wie oben definiert, besitzt, unter Verwendung eines Rhodium-Phosphin-Komplexes als Katalysator, dargestellt durch die folgende Formel (3-a) oder (3-b):
[Rh(L)(Y)(X)] (3-a)
[RH(L)(Y)](X) (3-b)
worin L eine Phosphinverbindung, dargestellt durch die folgende Formel (4): 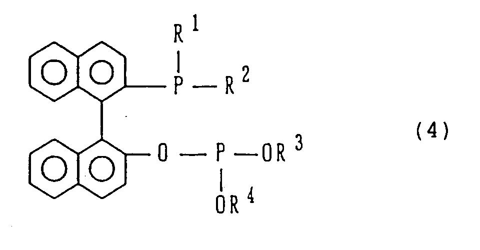 bedeutet, worin R¹ und R² gleich oder unterschiedlich voneinander sind und individuell eine Phenylgruppe, die durch ein Halogenatom oder eine C₁-C₆-Alkylgruppe substituiert sein kann, bedeuten, oder zusammen eine zweiwertige Kohlenwasserstoffgruppe bilden, R³ und R⁴, die gleich oder unterschiedlich voneinander sind, individuell eine C₁-C₆-Alkylgruppe oder eine Phenylgruppe, die durch ein Halogenatom, eine C₁-C₆-Alkylgruppe oder eine C₁-C₆-Alkoxygruppe substituiert sein kann, bedeuten, oder zusammen eine zweiwertige Kohlenwasserstoffgruppe bilden, Y Kohlenstoffmonoxid, einen neutralen Monodentat- oder Bidentatliganden mit niedrigerer Koordinationskraft als die von Kohlenstoffmonoxid, ein Olefin oder Dien bedeutet und X für ein Wasserstoffatom, ein einwertiges Anion oder Triphenylphosphin steht, oder X und Y zusammen ein β-Diketonat bedeuten.

2. Verfahren nach Anspruch 1, dadurch **gekennzeichnet,** daß die Vinylverbindung ausgewählt wird aus Vinylacetat, N-Vinylphthalimid, Vinylchlorid, Styrol, Acrylnitril, 4-Isobutylstyrol und 2-Methoxy-6-vinylnaphthalin.

3. Verfahren nach Anspruch 1, dadurch **gekennzeichnet,** daß das Binaphthylskelett der Phosphinverbindung in der Formel (3-a) oder (3-b) eine optisch aktive R- oder S-Struktur besitzt.

## Revendications

1. Procédé pour la production d'un aldéhyde optiquement actif représenté par la formule suivante (2):
OHC-CH(CH₃)-Q (2)
dans laquelle:
Q représente un atome d'halogène, un groupe alkyle en C₁₋₆, un groupe phtalimide, un groupe alkylcarbonyloxy en C₁₋₆, un groupe cyano, un groupe carboxy, un groupe alkyloxycarbonyle en C₁₋₆, un groupe alcoxy en C₁₋₆, un groupe alkylcarbonyle en C₁₋₆, un groupe acétylamino, un groupe benzoylamino, un groupe monoalkylamino, un groupe dialkylamino, un groupe benzoyle, un groupe phényle qui peut être substitué par un atome d'halogène, un groupe alkyle en C₁₋₆, ou un groupe alcoxy en C₁₋₆, ou un groupe naphtyle qui peut être substitué par un atome d'halogène, un groupe alkyle en C₁₋₆, ou un groupe alcoxy en C₁₋₆,
qui comprend l'hydroformylation d'un composé vinylique représenté par la formule suivante (1) :
CH₂=CH-Q (1)
dans laquelle Q est tel que défini plus haut, avec utilisation en tant que catalyseur, d'un complexe de rhodiumphosphine représenté par les formules (3-a) ou (3-b) suivantes:
[Rh(L)(Y)(X)] (3-a)
[Rh(L)(Y)](X) (3-b)
dans lesquelles L est un composé phosphine représenté par la formule suivante (4): 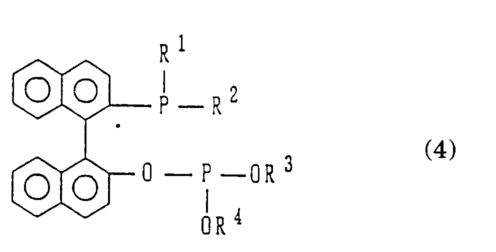 dans laquelle:
R¹ et R² sont identiques ou différents l'un de l'autre et représentent individuellement un groupe phényle qui peut être substitué par un atome d'halogène ou un groupe alkyle en C₁₋₆, ou représentent ensemble un groupe hydrocarboné divalent, R³ et R⁴ sont identiques ou différents l'un de l'autre et représentent individuellement un groupe alkyle en C₁₋₆, ou un groupe phényle qui peut être substitué par un atome d'halogène, un groupe alkyle en C₁₋₆ ou un groupe alcoxy en C₁₋₆, ou bien représentent ensemble un groupe hydrocarboné divalent,
Y est du monoxyde de carbone, un ligand neutre monodentate ou bidentate ayant une force de coordination inférieure à celle du monoxyde de carbone, une oléfine ou un diène, et
X représente un atome d'hydrogène, un anion monovalent ou une triphénylphosphine, ou bien X et Y représentent ensemble un β-dicétonate.

2. Procédé suivant la revendication 1, dans lequel le composé vinylique est choisi parmi l'acétate de vinyle, le N-vinylphtalimide, le chlorure de vinyle, le styrène, l'acrylonitrile, le 4-isobutylstyrène et le 2-méthoxy-6-vinylnaphtalène.

3. Procédé suivant la revendication 1, dans lequel le squelette binaphtyle du composé phosphine dans la formule (3-a) ou (3-b) a une structure R ou S optiquement active.
